# EUROPÄISCHE PATENTANMELDUNG

(11) **EP 2 017 757 A1**
(43) Veröffentlichungstag der Anmeldung: **21.01.2009**
(21) Anmeldenummer: 08158782.6
(22) Anmeldetag: 23.06.2008
(51) Int. Cl.: G06F 19/00

(54) **Anordnung für die Fernprogrammierung eines persönlichen medizinischen Gerätes**

(30) Priorität: 10.07.2007 DE 102007032469
(71) Anmelder: BIOTRONIK CRM Patent AG, 6341 Baar (CH)
(72) Erfinder: Dörr, Thomas, 12437, Berlin (DE); Reinke, Joachim, 10439, Berlin (DE)
(74) Vertreter: Lindner-Vogt, Karin L.

(57) **Zusammenfassung**

Die Erfindung besteht in einer Anordnung und einem Verfahren zur sicheren Fernprogrammierung eines Implantats. Dazu wird seitens eines Programmiergerätes eine TAN-Liste erzeugt und sowohl in dem Implantat (10) gespeichert als auch einem Arzt zur Verfügung gestellt. Die TAN-Liste ist vorzugsweise indiziert und das Implantat gibt jeweils den Index einer TAN vor, die ein Arzt eingeben muss, um einen Programmierauftrag durch das Implantat ausgeführt zu bekommen.

## Beschreibung

Die Erfindung betrifft eine Anordnung für die Fernprogrammierung eines persönlichen medizinischen Gerätes, insbesondere eines implantierbaren medizinischen Gerätes, wie beispielsweise einem Herzschrittmacher, einem Defibrillator oder dergleichen.

Im Zusammenhang mit Herzschrittmachern oder Defibrillatoren im Verbund mit einem Servicecenter ist es möglich und auch hinlänglich bekannt, seitens eines Herzschrittmachers oder Defibrillators gewonnene medizinische, physiologische oder Betriebsdaten zu dem zentralen Servicecenter zu übertragen, um diese Daten dort auszuwerten und über eine entsprechende Benutzerschnittstelle einem betreuenden Arzt zur Verfügung zu stellen.

Einige Funktionen solcher Implantate sind durch Software oder Firmenware gesteuert und daher programmierbar. Dabei kommt es immer wieder vor, dass nach anfänglicher Programmierung kurz vor, während oder nach der Implantation weitere Programmierungen oder Umprogrammierungen wünschenswert sind, um das Implantat besser auf möglicherweise inzwischen veränderte Gesundheitszustände eines Patienten einstellen zu können oder die Leistungsfähigkeit des Implantats anderweitig zu erhöhen. Häufig geschieht ein derartiges Programmieren oder Umprogrammieren dadurch, dass ein Arzt zu einem jeweiligen Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantats kann jedoch grundsätzlich auch aus der Ferne, beispielsweise über das zentrale Servicecenter, geschehen. Hierzu kann eine Datenverbindung zwischen dem Servicecenter und einem Patientengerät hergestellt werden, welches sich üblicherweise in der Nähe eines Patienten befindet und eine bidirektionale Datenverbindung zwischen dem Implantat und dem Patientengerät herstellen kann. Die Verbindung zwischen Servicecenter und Patientengerät kann dabei drahtlos separat gebunden, beispielsweise über das Telefonnetz, das Internet oder ähnliche Datenleitungen, ausgebildet sein.

Hierbei besteht grundsätzlich das Problem, sicherzustellen, dass das jeweilige Implantat oder auch das Patientengerät als persönliches medizinisches Gerät nicht infolge einer fehlerhaften Datenübertragung oder gar missbräuchlich umprogrammiert werden kann.

Zur Lösung dieses Problems ist es beispielsweise gemäß US 6,442,432 vorgesehen, Programmieraufträge für das Programmieren oder Umprogrammieren eines Implantats mit einem Public-Key-Verschlüsselungsverfahren, wie beispielsweise PGP, zu verschlüsseln beziehungsweise zu verifizieren.

Der vorliegenden Erfindung liegt die Aufgabe zugrunde, auf alternative Weise die Verifikation sicherzustellen, damit ein Programmierauftrag an ein persönliches Gerät, wie ein Implantat oder ein Patientengerät, in jedem Fall authentisch ist und von befugter Quelle stammt.

Erfindungsgemäß wird diese Aufgabe durch eine Anordnung der eingangs genannten Art gelöst, welche als Komponenten neben dem persönlichen Gerät ein dem persönlichen Gerät zugeordnetes Programmiergerät aufweist, das einen TAN-Listen-Generator enthält und ausgebildet ist, eine Liste mit Transaktionsnummern (TAN) zu generieren und diese TAN-Liste an das persönliche Gerät und an ein weiteres, für einen Arzt zugängliches Gerät zu versenden oder direkt auszudrucken.

Die Anordnung umfasst somit ein programmierbares persönliches Gerät, das insbesondere ein aktives medizinisches Implantat wie ein Herzschrittmacher oder ein Kardioverter/Defibrillator ist, sowie ein Programmiergerät und ein zentrales Servicecenter. Das programmierbare persönliche Gerät kann direkt oder beispielsweise über eine kurzreichweitige, drahtlose Datenkommunikationsverbindung mit Hilfe des Programmiergerätes programmiert werden, oder aus der Ferne über das Servicecenter. Hierzu besitzt das programmierbare persönliche Gerät eine erste Datenkommunikationsschnittstelle zum wenigstens mittelbaren Verbinden des persönlichen programmierbaren Gerätes mit dem Servicecenter, sowie eine zweite Datenkommunikationsschnittstelle, die von der ersten Datenkommunikationsschnittstelle verschieden ist und ausgebildet ist, eine direkte, drahtlose Datenkommunikation zwischen dem persönlichen Gerät und dem Programmiergerät zu ermöglichen.

Das Servicecenter besitzt eine Datenkommunikationsschnittstelle zum wenigstens mittelbaren Verbinden des Servicecenters mit dem persönlichen Gerät und eine Benutzerschnittstelle, die ausgebildet ist, Programmieraufträge für das programmierbare persönliche Gerät entgegenzunehmen, damit der Programmierauftrag über das Servicecenter an das persönliche Gerät übertragen werden kann.

Das Programmiergerät weist wenigstens eine Datenkommunikationsschnittstelle auf, die mit der zweiten Datenkommunikationsschnittstelle des persönlichen Gerätes kompatibel ist und es erlaubt, eine direkte, drahtlose Datenkommunikation zwischen dem Programmiergerät und dem persönlichen Gerät durchzuführen.

Erfindungsgemäß besitzt das Programmiergerät einen TAN-Listen-Generator, der ausgebildet ist, eine TAN-Liste zu generieren und zum einen an das persönliche Gerät und zum anderen entweder an einen TAN-Server als weitere Komponente der Anordnung oder an ein persönliches Kommunikationsgerät eines Arztes, beispielsweise ein Mobiltelefon, ein Faxgerät oder einen E-Mailclient zu übertragen oder auf einem angeschlossenen Drucker auszudrucken.

Insoweit ist das Servicecenter noch kein notwendiger Bestandteil der Anordnung, sondern kommt erst dann zum Einsatz, wenn das persönliche Gerät aus der Ferne programmiert werden soll, wobei Programmieraufträge mit einer TAN gesichert werden. Dies geschieht dadurch, dass das Servicecenter zu jedem Programmierauftrag die Eingabe einer TAN anfordert und dem Programmierauftrag hinzufügt. Anschließend übersendet das Service-center den Programmierauftrag zusammen mit der TAN an das persönliche Gerät. Das persönliche Gerät ist dazu ausgebildet, die nächste in seinem Speicher gespeicherte TAN mit derjenigen TAN zu vergleichen, die einem jeweiligen erhaltenen Programmierauftrag beigefügt ist. Das persönliche Gerät ist dann weiterhin dazu ausgebildet, einen Programmierauftrag nur auszuführen, wenn die beiden TAN übereinstimmen und andernfalls den Programmierauftrag zu ignorieren oder zu löschen.

Vorzugsweise ist der TAN-Listen-Generator des Programmiergerätes dazu ausgebildet, zu jeder generierten TAN einen zugehörigen Index zu bestimmen, gemeinsam iTAN genannt, und TAN und Index einer Liste hinzuzufügen.
In einer weiteren Ausführungsform ist das Programmiergerät dazu ausgebildet, TANs ohne einen Index zu generieren und die TAN ebenfalls einer Liste hinzuzufügen. Die so entstandene Liste (TAN-Liste) kann somit iTANs, TANs oder beides enthalten.

Das Programmiergerät ist weiter dazu ausgebildet, die so erzeugte TAN-Liste oder iTAN-Liste entweder an einen TAN-Server oder an ein persönliches Kommunikationsgerät wie Mobiltelefon, Faxgerät oder E-Mailclient zu übertragen oder auszudrucken.

Beim Verifizieren mit iTAN-Listen ist es in diesem Zusammenhang vorteilhaft, wenn das persönliche Gerät, also beispielsweise das Implantat, dazu ausgebildet ist, nach Erhalt einer iTAN-Liste oder nach dem Ausführen eines Programmierauftrags einen Index für einen nachfolgenden Programmierauftrag zu bestimmen und an das Servicecenter zu übermitteln.

Vorzugsweise ist das Servicecenter in Verbund mit seiner Servicebenutzerschnittstelle ausgebildet, zu jedem Programmierauftrag eine iTAN abzufragen, so dass ein Arzt aufgefordert ist, die zu dem Index gehörende TAN einzugeben. Das Servicecenter fügt die TAN dem Programmierauftrag bei und überträgt den Programmierauftrag zu dem persönlichen Gerät. Falls der Arzt die richtige TAN bzw. die, zu dem angegebenen Index gehörende TAN angegeben hat, kann das persönliche Gerät den Programmierauftrag ausführen. Andernfalls wird der Programmierauftrag ignoriert.

Die Benutzerschnittstelle ist hierbei vorzugsweise als eine vom Servicecenter entfernt lauffähige Fernprogrammieranwendung ausgebildet und mit dem Servicecenter verbunden. Dies erlaubt es, die Fernprogrammierung eines Implantats von einem häuslichen Rechner eines Arztes aus über das Servicecenter vorzunehmen.

Vorteilhafterweise weist die Anordnung als weitere Komponente einen physikalisch separaten und räumlich entfernten TAN-Server auf. Das Programmiergerät besitzt dann neben der Datenkommunikationsschnittstelle für den direkten Datenaustausch mit dem persönlichen Gerät eine weitere Datenkommunikationsschnittstelle, um eine TAN-Liste an den TAN-Server übertragen zu können. Der TAN-Server weist vorzugsweise eine Datenbank auf, die einander zugeordnete Einträge für eine Benutzerkennung, ein der Benutzerkennung zugeordnetes persönliches Gerät, gekennzeichnet durch seine Gerätekennung und eine der Benutzerkennung zugeordnete Nachrichtenadresse eines adressierbaren Kommunikationsgerätes enthält. Weiterhin besitzt der TAN-Server eine TAN-Benutzerschnittstelle die ausgebildet ist, Eingaben eines Benutzers entgegenzunehmen und die Eingaben einer Benutzerkennung zuzuordnen und derart zugeordnet an den TAN-Server weiterzuleiten. Weiterer Bestandteil des TAN-Servers ist vorzugsweise eine Nachrichten-schnittstelle, über die der TAN-Server eine TAN an ein adressierbares Kommunikationsgerät wie ein Mobiltelefon, einen E-Mailclient oder ein Faxgerät versenden kann, welches durch seine Nachrichtenadresse identifiziert ist. Schließlich besitzt der TAN-Server wenigstens eine Datenkommunikationsschnittstelle, um TAN-Listen vom Programmiergerät empfangen zu können.

Der TAN-Server ist ausgebildet, eine einer Benutzerkennung zugeordnete TAN-Anfrage zu empfangen und anschließend eine TAN-Nachricht zu generieren und über die Nachrichtenschnittstelle an das durch die über die Datenbankeinträge der Benutzerkennung des anfragenden Benutzers zugeordnete adressierbare Kommunikationsgerät zu senden. Der TAN-Server erlaubt es somit, dass ein Arzt eine von dem Programmiergerät erzeugte TAN-Liste nicht selbst aufbewahren und verwalten muss. Vielmehr geschieht dies über den TAN-Server.

Im Falle einer bevorzugten Ausführungsvariante mit indizierten TAN enthält die TAN-Anfrage an den TAN-Server neben der Benutzerkennung und der Gerätekennung zusätzlich den zuletzt von dem persönlichen Gerät bestimmten Index für die einzugebene TAN.

Der TAN-Server ist vom Servicecenter administrativ und vorzugsweise räumlich völlig getrennt.

Die der Erfindung zugrunde liegende Aufgabe wird im Übrigen ebenfalls durch ein im Sinne der erfindungsgemäßen Anordnung ausgestaltetes Programmiergerät, also konkret ein Programmiergerät mit TAN-Listen-Generator und durch ein ebenfalls im Sinne der Erfindung ausgestaltetes persönliches Gerät gelöst. Das persönliche Gerät ist insbesondere ein aktives medizinisches Implantat, das dazu ausgebildet ist, TAN-Listen samt Indices zu speichern und rechtzeitig vor einem neuen Programmierauftrag den Index einer TAN zu bestimmen, die dann dem Programmierauftrag hinzuzufügen ist, damit das persönliche Gerät den Programmierauftrag auch ausführt.

Weiterhin wird die der Erfindung zugrunde liegende Aufgabe durch ein Verfahren gelöst, welches die folgenden Schritte aufweist:
- Bereitstellen eines Implantats (10) und eines dem Implantat für die Dauer des Programmiervorgangs eindeutig zugeordneten Programmiergerätes (70)
- Generieren einer TAN-Liste mit zugehörigen Indices oder ohne Indices durch das Programmiergerät (70)
- Übertragen der TAN-Liste mit Indices oder ohne Indices an das Implantat (10) und an ein drittes Gerät
- Bei TAN-Listen mit Indices Bestimmen eines Indexes durch das Implantat
- Zusammenstellen eines Programmierauftrags
- Bei TAN-Listen mit Indices Abfragen einer TAN unter Anzeige des vom Implantat bestimmten Index
- Hinzufügen einer TAN zu dem Programmierauftrag und Übertragen des Programmierauftrags an das Implantat
- Bei TAN-Listen mit Indices Vergleich des im Implantat gespeicherten, zu dem vom Implantat zuletzt bestimmten Index gehörenden TAN mit der im Programmierauftrag enthaltenen TAN
- Ausführen des Programmierauftrags, falls die TAN identisch sind, oder
- Ignorieren oder Löschen des Programmierauftrags, falls die TAN nicht identisch sind.

Vorteilhafterweise kann ein weiterer Schritt,
- Rückmeldung über Erfolg oder Misserfolg der Ausführung des Programmierauftrags dazukommen.

Weitere vorteilhafte Ausgestaltungsvarianten ergeben sich durch Kombination der Merkmale verschiedener Ansprüche oder aus der nachfolgenden Beschreibung eines Ausführungsbeispiels.

Die Erfindung soll nun anhand eines Ausführungsbeispiels mit Bezug auf die Figuren näher erläutert werden. Diese zeigen in
- Figur 1:: Eine Übersicht über eine Anordnung zur Fernprogrammierung eines Implantats;
- Figur 2:: Blockschaltbilder der Komponenten der Anordnung aus Figur 1
- Figur 3:: das zur Aktivierung der Fernprogrammierung; und
- Figur 4:: den Ablauf einer Fernprogrammierung.

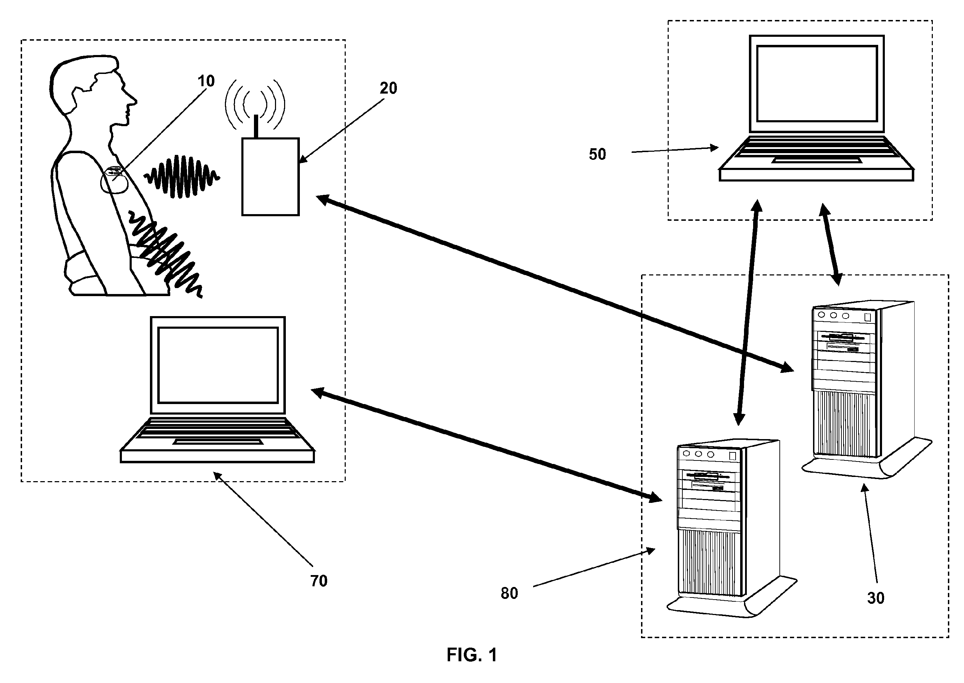

Figur 1 zeigt die Komponenten einer beispielhaften Anordnung zur Fernprogrammierung von Implantaten.

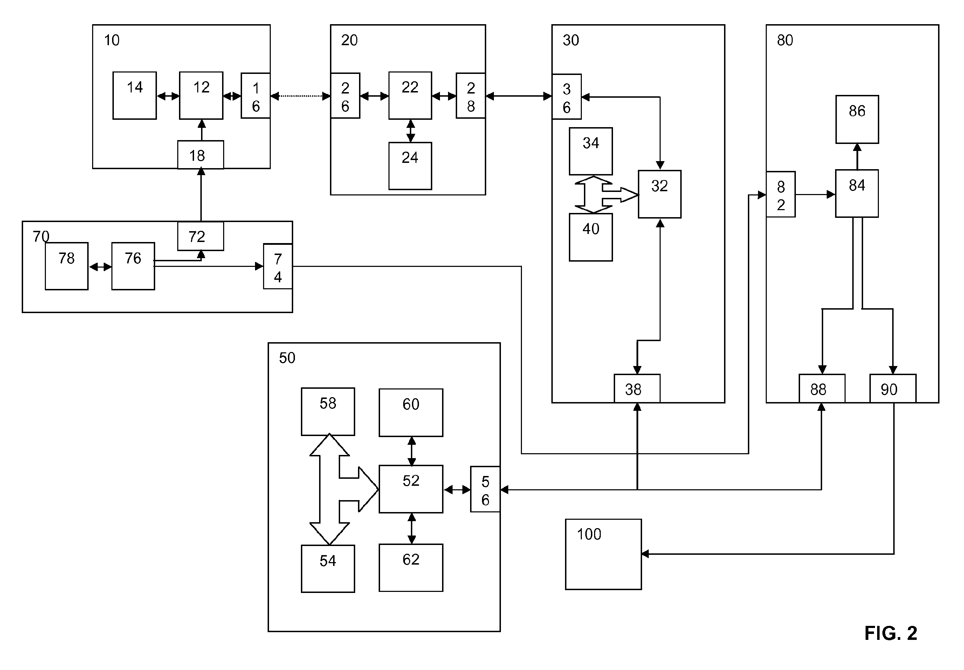

Eine Komponente ist das Implantat 10 selbst. Dieses besitzt eine Steuerung 12 (siehe Figur 2) die sowohl mit einem Speicher 14 als auch mit einer Telemetrieeinheit 16 verbunden ist. Die Telemetrieeinheit 16 ermöglicht eine drahtlose, bidirektionale Datenübertragung von und zu dem sie enthaltenden Implantat 10.

Eine weitere Komponente ist ein Patientengerät 20, dass ebenfalls eine Steuerung 22 aufweist, die mit einem Speicher 24 und einer ersten Telemetrieeinheit 26 für eine bidirektionale, Datenübertragung von und zu dem Implantat 10 verbunden ist, sowie mit einer zweiten Datenkommunikationsschnittstelle 28, über die das sie enthaltende Patientengerät 20 bidirektional Daten mit einem zentralen Servicecenter 30 austauschen kann.

Das zentrale Servicecenter 30 weist neben einer Steuerung 32 eine zentrale Datenbank 34 und eine erste Datenkommunikationsschnittstelle 36 auf, über die das Servicecenter 30 bidirektional Daten mit dem Patientengerät 20 austauschen kann. Außerdem weist das zentrale Servicecenter 30 eine Fernprogrammierservereinheit 40 auf, die sowohl mit der Steuereinheit 32 des Servicecenters verbunden ist als auch mit einer Internetschnittstelle 38 zum Anschluss eines Fernprogrammierclients 50.

Der Fernprogrammierclient 50 besitzt ebenfalls eine zentrale Steuereinheit 52, die mit einem Speicher 54 und einer Datenkommunikationsschnittstelle 56 für eine bidirektionale Datenkommunikation mit dem Service Center 30 verbunden ist. Außerdem weist der Fernprogrammierclient 50 eine Fernprogrammieranwendung 58 auf, die mit Hilfe der zentralen Steuereinheit 52 und des Speichers 54 ausgeführt werden kann und entweder als stand-alone Lösung ausschließlich auf den Fernprogrammierclient 50 lauffähig ist oder als Serveranwendung teilweise oder ganz auch auf das zentrale Servicecenter 30 und deren Fernprogrammierservereinheit 40 zurückgreift.

Die Fernprogrammieranwendung 58 erlaubt es, auf den Fernprogrammierclient 50 Programme zusammenzustellen, die für das Implantat 10 und dessen Steuerung 12 ausführbar sind. Die so zusammengestellten Programme können in Form eines Programmierauftrages von dem zentralen Servicecenter 30 über das Patientengerät 20 zum Implantat 10 übertragen werden.

Um von der Fernprogrammieranwendung generierte Darstellungen anzuzeigen, weist der Fernprogrammierclient 50 eine Anzeige 60 auf. Damit ein Benutzer, beispielsweise ein Arzt Eingaben für die Fernprogrammierung des Implantats 10 machen kann ist außerdem eine Eingabeeinheit 62 vorgesehen.

Räumlich befinden sich das Implantat 10 und das Patientengeräts 20 in einem bzw. in der Nähe eines Patienten. Das zentrale Servicecenter 30 ist an einem zentralen Ort angeordnet. Der Fernprogrammierclient 50 befindet sich in der Nähe eines Arztes und kann vom Servicecenter 30 weit entfernt sein.

Erfindungsgemäß weist die Anordnung außerdem ein Programmiergerät 70 auf, das eine erste Datenkommunikationsschnittstelle 72 besitzt, mit der das Programmiergerät 70 eine kurzreichweitige, drahtlose Datenkommunikation mit dem Implantat 10 durchführen kann. Darüber hinaus weist das Programmiergerät 70 eine zweite Datenkommunikationsschnittstelle 74 auf, über die das Programmiergerät 70 beispielsweise mit einem TAN-Server 80 oder alternativ auch mit dem Servicecenter 30 oder dem Fernprogrammierclient 50 verbunden werden kann. Das Programmiergerät 70 besitzt neben den üblichen Komponenten eines derartigen Programmiergerätes eine Steuerung 76 und einen TAN-Listen-Generator 78.

Mittels des TAN-Listen-Generators 78 kann das Programmiergerät indizierte oder nicht-indizierte Listen von Transaktionsnummern (iTAN / TAN) generieren und über die Steuerung 76 und die Datenkommunikationsschnittstelle 72 zum einen an das Implantat 10 übertragen und zum anderen beispielsweise an den TAN-Server 80.

Hier ist anzumerken, dass das Implantat 10 neben der Datenkommunikationsschnittstelle 16 für die Datenkommunikation mit dem Patientengerät 20 eine Datenkommunikation zur Schnittstelle 18 aufweist, die der kurzreichweitigen Datenkommunikation zwischen Programmiergerät 70 und Implantat 10 dient. Über diese Datenkommunikationsschnittstelle 18 kann das Implantat 10 eine indizierte TAN-Liste empfangen und in seinem Speicher 14 speichern.

Die indizierte oder nicht-indizierte TAN-Liste wird seitens des Programmiergerätes 70 au-βerdem zu dem TAN-Server 80 übertragen. Hierzu besitzt der TAN-Server 80 einen Eingang 82, der mit einer Steuerung 84 des TAN-Servers 80 verbunden ist. Die Steuerung 84 des TAN-Servers 80 ist wiederum mit einem Speicher 86 des TAN-Servers 80 verbunden. Dieser Speicher umfasst eine Datenbank, die Einträge für eine seitens des Programmiergerätes 70 empfangene TAN-Liste sowie diesen Einträgen zugeordnet Einträge für eine Benutzerkennung, eine Gerätekennung und eine Nachrichtenadresse aufweist. Über die Benutzerkennung ist ein Benutzer identifiziert. Die Gerätekennung kennzeichnet das Implantat 10 und die Nachrichtenadresse ist die Adresse eines persönlichen Kommunikationsgerätes wie beispielsweise eines Mobiltelefons, eines Faxgerätes oder eines E-Mailclients. Über einen Ausgang 88 kann der TAN-Server 80 mit dem Fernprogrammierclient 50 verbunden werden.

Ein zweiter Ausgang 90 dient dem TAN-Server 80 dazu, Nachrichten an ein adressierbares Kommunikationsgerät 100 wie beispielsweise einem Faxgerät, einem Mobiltelefon o.ä. zu senden, die jeweils durch die Nachrichtenadressen identifiziert sind.

Mit Hilfe einer entsprechenden Anwendung auf dem Fernprogrammierclient 50 kann ein Benutzer eine im Speicher 86 des TAN-Servers 80 gespeicherte TAN-Liste abrufen. Dazu muss der Benutzer seine Benutzerkennung eingeben sowie die Gerätekennung des zu programmierenden Implantats, da die TAN-Listen implantatspezifisch sind. Zuvor hat sich der Benutzer auf dem TAN-Server 80 akkreditiert, so dass der TAN-Server 80 nun eine Liste mit TAN an das Kommunikationsgerät sendet, das über die Zuordnung der Nachrichtenadresse zu den übrigen Einträgen in der Datenbank dem Benutzer zugeordnet ist.

Um das Implantat 10 fern zu programmieren, verlangt die Fernprogrammieranwendung 58 neben der Eingabe eines Programmierauftrags auch die Eingabe einer TAN, die demjenigen Index entspricht, den das Implantat 10 vorgegeben hat. Dieser Index wird von dem Implantat 10 bestimmt und über das Patientengerät 20 zum Servicecenter 30 übertragen, und von diesem auf die Fernprogrammieranwendung 58.

Der Benutzer muss nun nach Fertigstellen eines Programmierauftrags die entsprechende TAN eingeben und kann diese beispielsweise durch Abrufen einer TAN vom TAN-Server 80 erhalten oder einem zuvor mit dem Programmiergerät 70 erstellten Ausdruck entnehmen. Wenn die so dem Programmierauftrag hinzugefügte TAN der in dem Implantat 10 gegebenenfalls unter dem entsprechenden Index gespeicherten TAN entspricht, kann das Implantat den Programmierauftrag nach Empfang ausführen. Andernfalls wird das Implantat den Programmierauftrag ignorieren.

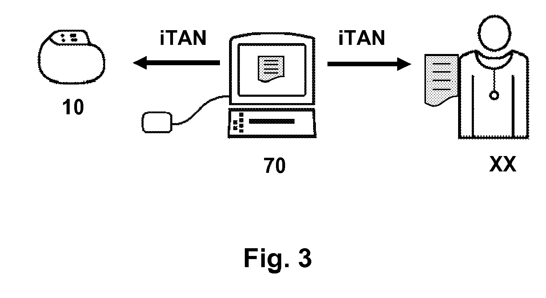

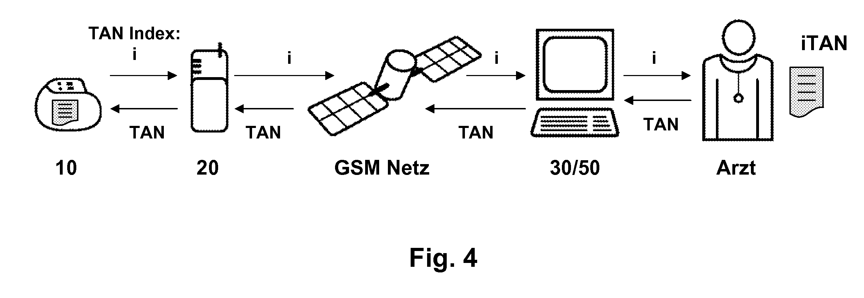

Die Vorgänge zum Erzeugen einer TAN-Liste (Fig. 3) und Fernprogrammierung (Fig. 4) sind in die nachfolgenden Figuren 3 bzw. 4 noch einmal schematisch erläutert

In Figur 3 ist das Verfahren zur Aktivierung der Fernprogrammierung dargestellt. In dem Programmiergerät (70) ist ein TAN/iTAN-Server integriert, der Transaktionsnummern und indizierte Transaktionsnummern (TAN/iTAN) generieren kann. Dieser Server überträgt auf Anforderung des Arztes (200) über den Fernprogrammierserver (50) eine indizierte Liste mit TAN-Nummern an das elektronische Implantat (10) und stellt diese Liste nach erfolgreicher Übertragung zum elektronischen Implantat dem Arzt in Form eines Ausdrucks und optional als Datei zur Verfügung.

In Figur 4 ist der Ablauf einer Fernprogrammierung dargestellt. Das elektronische Implantat 10 übermittelt mittels regulärer Datenübertragung den Index der nächsten zur Fernprogrammierung benötigten TAN aus der gespeicherten Liste via Patientengerät 20 und Netzwerkdienstleister (z.B. GSM Provider) zum Service Center 30. Sofern der Arzt eine Fernprogrammierung veranlasst, wird dieser vom Service Center aufgefordert, die TAN mit dem Index i von der TAN-Liste einzugeben. Diese TAN wird dann gemeinsam mit dem Programmparametern als Bestandteil eines Programmierauftrags für die Fernprogrammierung zum elektronischen Implantat übermittelt.

Das elektronische Implantat 10 prüft die übermittelte TAN und akzeptiert die Fernprogrammierung nur dann, wenn die übermittelte TAN der im Implantat gespeicherten TAN mit dem Index i entspricht. Des Weiteren wird die "verbrauchte" TAN mit dem Index i als verbraucht markiert und mittels Zufallsgenerator oder einem gleichwertigen anderen Verfahren im elektronischen Implantat 10 der nächste TAN-Index bestimmt und dem Service Center 30 mitgeteilt.

Sind alle TANs verbraucht wird die Fernprogrammierung im elektronischen Implantat 10 deaktiviert und dieser Zustand dem Service Center 30 mitgeteilt. Die Reaktivierung der Fernprogrammierung ist dann nur über den Mechanismus aus Figur 3 möglich.

## Patentansprüche

1. Anordnung für die Fernprogrammierung eines programmierbaren persönlichen medizinischen Gerätes (10), insbesondere eines implantierbaren medizinischen Gerätes wie ein Herzschrittmacher, Defibrillator oder dergleichen, mit den Komponenten:
- programmierbares persönliches Gerät (10)
- Programmiergerät (70)
von denen das programmierbare persönliche Gerät (10)
eine erste Datenkommunikationsschnittstelle (28) zum wenigstens mittelbaren Verbinden des persönlichen programmierbaren Gerätes (10) mit einem Servicecenter (30) und
eine zweite, von der ersten verschiedene Datenkommunikationsschnittstelle (26) zum direkten, drahtlosen Verbinden des programmierbaren persönlichen Gerätes (10) mit dem Programmiergerät (70) aufweist, und
von denen das Programmiergerät (70)
eine Datenkommunikationsschnittstelle (72) zum direkten, drahtlosen Verbinden des Programmiergerätes (70) mit dem programmierbaren persönlichen Gerät (10) aufweist
**dadurch gekennzeichnet, dass**
das Programmiergerät (70) einen TAN-Listen-Generator (78) aufweist, der ausgebildet ist, eine TAN-Liste zu generieren und zum einen an das programmierbare persönliche Gerät (10) und zum anderen entweder an einen TAN-Server (80) oder an ein persönliches Kommunikationsgerät (100) zu übertragen oder auszudrucken.

2. Anordnung nach Anspruch 1, **dadurch gekennzeichnet, dass** die Anordnung als weitere Komponente ein Service-Center (30) aufweist, das eine Datenkommunikationsschnittstelle (36) für eine Datenkommunikation mit dem persönlichen Gerät (10) aufweist sowie
eine weitere, mit dem Service Center (30) verbundene Service-Benutzschnittstelle (38) besitzt,
- die es erlaubt, Programmieraufträge für das programmierbare persönliche Gerät (40) zusammenzustellen,
- ein Absenden eines zusammengestellten Programmierauftrags auszulösen und
- die ausgebildet ist, eine manuelle Eingabe einer TAN zu einem Programmierauftrag abzufragen und eine entgegengenommene TAN einem Programmierauftrag hinzuzufügen.,
und das ausgebildet ist,
einen abgesendeten Programmierauftrag zusammen mit einer TAN an das persönliche Gerät (10) zu senden,
wobei das persönliche Gerät (10) dazu ausgebildet ist, eine seitens des Programmiergerätes (70) empfangene, gespeicherte TAN mit einer zusammen mit einem Programmierauftrag seitens des Servicecenters (30) empfangenen TAN zu vergleichen und den Programmierauftrag nur auszuführen, wenn beide TAN identisch sind.

3. Anordnung nach Anspruch 1 oder 2, **dadurch gekennzeichnet, dass** der TAN-Listen-Generator (78) des Programmiergerätes (70) ausgebildet ist, zu jeder generierten TAN einen zugehörigen Index zu generieren und zusammen mit jeder TAN zum einen an das persönliche programmierbare Gerät (10) und zum anderen entweder an einen TAN-Server (80) oder an ein adressierbares Kommunikationsgerät (100) zu übertragen oder zusammen mit der TAN-Liste auszudrucken.

4. Anordnung nach Anspruch 3, **dadurch gekennzeichnet, dass** das persönliche Gerät (10) ausgebildet ist, nach Erhalt einer TAN-Liste mit zugehörigen Indices oder nach Ausführen eines Programmierauftrags einen Index einer TAN für einen nachfolgenden Programmierauftrag zu bestimmen und an das Servicecenter (30) zu übermitteln.

5. Anordnung nach den Ansprüchen 2 und 4, **dadurch gekennzeichnet, dass** das Servicecenter (30) in Verbund mit der Service-Benutzschnittstelle (38) ausgebildet ist, eine TAN zu einem Programmierauftrag abzufragen und hierzu den Index der einzugebenden TAN anzuzeigen.

6. Anordnung nach Anspruch 4 oder 5, **dadurch gekennzeichnet, dass** die Service-Benutzerschnittstelle (38) als auf einem vom Service Center (30) entfernt lauffähige Fernprogrammieranwendung ausgebildet oder mit einer solchen verbunden ist.

7. Anordnung nach einem der Ansprüche 1 bis 6, **dadurch gekennzeichnet, dass** die Anordnung als weitere Komponente einen TAN-Server (80) aufweist und das Programmiergerät (70) eine weitere Datenkommunikationsschnittstelle (74) aufweist, die ausgebildet ist, eine Datenübertragung von dem Programmiergerät (70) zu dem TAN-Server (80) zu erlauben und ausgebildet ist, eine generierte TAN-Liste an den TAN-Server (80) zu übertragen.

8. Anordnung nach Anspruch 7, **dadurch gekennzeichnet, dass** der TAN-Server (80) aufweist:
eine Datenbank (86), die einander zugeordnete Einträge für eine Benutzer-Kennung, ein der Benutzer-Kennung zugeordnetes persönliches Gerät (10), **gekennzeichnet durch** eine Geräte-Kennung und eine der Benutzer-Kennung zugeordnete Nachrichten-Adresse eines adressierbaren Kommunikationsgerätes (100) enthält,
eine TAN-Benutzerschnittstelle, die ausgebildet ist Eingaben eines Benutzers entgegenzunehmen und die Eingaben einer Benutzer-Kennung zuzuordnen und derart zugeordnet an den TAN Server (80) weiterzuleiten,
eine Nachrichten-Schnittstelle (90) über die der TAN-Server (80) eine Nachricht an ein adressierbares Kommunikationsgerät (100) eines Benutzers, dass **durch** eine Nachrichten-Adresse identifiziert ist, senden kann und eine Datenkommunikationsschnittstelle (82) für eine wenigstens mittelbare Verbindung zum Empfang von Daten seitens des Programmiergerätes (70)
und ausgebildet ist:
zum Zwecke der TAN-Zurverfügungstellung eine einer Benutzerkennung zugeordnete TAN-Anfrage zu empfangen und
anschließend eine eine TAN enthaltende Nachricht zu generieren und über die Nachrichtenschnittstelle (90) an das **durch** die über die Datenbank-Einträge der Benutzer-Kennung des abfragenden Benutzers zugeordnete adressierbare Kommunikationsgerät (100) zu senden.

9. Anordnung nach einem der Ansprüche 3 bis 6 und Anspruch 7 oder 8, **dadurch gekennzeichnet, dass** die Service-Benutzerschnittstelle ausgebildet ist, eine TAN-Anfrage eines Benutzers zusammen mit einer Benutzerkennung dieses Benutzers entgegenzunehmen, wobei das Service-Center (30) eine zweite Datenkommunikationsschnittstelle (38) aufweist, die ausgebildet ist, einen Datenaustausch vom Servicecenter (30) zum TAN-Server (80) zu ermöglichen und das Service Center (30) ausgebildet ist, eine über die Service-Benutzerschnittstelle empfangene TAN-Anfrage an den TAN-Server (80) weiterzuleiten.

10. TAN-Server für eine Anordnung gemäß der Ansprüche 7 bis 9, **dadurch gekennzeichnet, dass** der TAN-Server (80)
eine Datenbank aufweist, die einander zugeordnete Einträge für eine Benutzer-Kennung, ein der Benutzer-Kennung zugeordnetes persönliches Gerät (10), **gekennzeichnet durch** eine Geräte-Kennung und eine der Benutzer-Kennung zugeordnete Nachrichten-Adresse eines adressierbaren Kommunikationsgerätes (100) enthält, sowie
eine TAN-Benutzerschnittstelle, die ausgebildet ist, Eingaben eines Benutzers entgegenzunehmen und die Eingaben einer Benutzer-Kennung zuzuordnen und derart zugeordnet als TAN-Anfrage an den TAN-Server (80) weiterzuleiten, eine Nachrichten-Schnittstelle (90) über die der TAN-Server eine Nachricht an ein adressierbares Kommunikationsgerät (100) eines Benutzers, das **durch** eine Nachrichten-Adresse identifiziert ist, senden kann
und ausgebildet ist:
zum Zwecke der TAN-Zurverfügungstellung
eine einer Benutzerkennung zugeordnete TAN-Anfrage zu empfangen und
anschließend eine eine TAN enthaltende Nachricht zu generieren und über die Nachrichtenschnittstelle an das **durch** die über die Datenbank-Einträge der Benutzer-Kennung des abfragenden Benutzers zugeordnete adressierbare Kommunikationsgerät (100) zu versenden.

11. Programmiergerät für eine Anordnung gemäß eines der Ansprüche 1 bis 9, **dadurch gekennzeichnet, dass** das Programmiergerät (70) eine Datenkommunikationsschnittstelle (72) zum direkten, drahtlosen Verbinden des Programmiergerätes (70) mit einem persönlichen programmierbaren Geräte (10), insbesondere einen implantierbaren Herzschrittmacher und/oder Kardioverter/Defibrillator aufweist
**dadurch gekennzeichnet, dass**
das Programmiergerät (70) einen TAN-Listen-Generator aufweist, der ausgebildet ist, eine TAN-Liste zu generieren und zum einen an das persönliche programmierbare Gerät (10) und zum anderen entweder an einen TAN-Server (80) oder an ein adressierbares Kommunikationsgerät (100) zu übertragen.

12. Programmiergerät nach Anspruch 11, **dadurch gekennzeichnet, dass** das Programmiergerät (70) eine weitere Datenkommunikationsschnittstelle enthält, die ausgebildet ist, eine Datenübertragung von dem Programmiergerät zu dem TAN-Server (80) zu erlauben und außerdem ausgebildet ist, eine generierte TAN-Liste an den TAN-Server (80) zu übertragen.

13. Persönliches Gerät für eine Anordnung gemäß eines der Ansprüche 1 bis 9, **dadurch gekennzeichnet, dass** ein persönliches Gerät (10) ein aktives medizinisches Implantat, insbesondere ein implantierbarer Herzschrittmacher oder Kardioverter/Defibrillator sein kann, das neben wenigstens einem Stimulationsimpulsgenerator eine programmierbare Steuerung und einen Speicher zum Speichern einer TAN-Liste aufweist sowie eine erste Datenkommunikationsschnittstelle (16) zum wenigstens mittelbaren Verbinden des Implantats (10) mit dem Service Center (30) und eine zweite, von der ersten verschiedene Datenkommunikationsschnittstelle (18) zum direkten, drahtlosen Verbinden des Implantats mit dem Programmiergerät (70) aufweist und ausgebildet ist, über die zweite Datenkommunikationsschnittstelle (18) eine TAN-Liste zu empfangen und in dem Speicher zu speichern, und über die erste Datenkommunikationsschnittstelle (16) Programmieraufträge zu empfangen und nach Empfang eines Programmierauftrags eine gespeicherte TAN mit einer zusammen mit dem Programmierauftrag empfangenen TAN zu vergleichen und den Programmierauftrag nur auszuführen, wenn beide TAN identisch sind.

14. Persönliches Gerät nach Anspruch 13, **dadurch gekennzeichnet, dass** das persönliche Gerät (10) ausgebildet ist, eine Liste von TAN mit jeweils zugehörigem Index zu speichern und nach Erhalt einer TAN-Liste mit zugehörigen Indices oder nach Ausführen eines Programmierauftrags einen Index einer TAN für einen nachfolgenden Programmierauftrag zu bestimmen und über die erste Datenkommunikationsschnittstelle zu versenden.

15. Verfahren zum sicheren Programmieren eines medizinischen Implantats mit einer Anordnung gemäß eines der Ansprüche 1 bis 9, mit den Verfahrensschritten:
- Bereitstellen eines Implantats (10) und eines dem Implantat für die Dauer des Programmiervorgangs eindeutig zugeordneten Programmiergerätes (70)
- Generieren einer TAN-Liste mit zugehörigen Indices oder ohne Indices durch das Programmiergerät (70)
- Übertragen der TAN-Liste mit Indices oder ohne Indices an das Implantat (10) und an ein drittes Gerät
- Bei TAN-Listen mit Indices Bestimmen eines Indexes durch das Implantat
- Zusammenstellen eines Programmierauftrags
- Bei TAN-Listen mit Indices Abfragen einer TAN unter Anzeige des vom Implantat bestimmtes Indexes
- Hinzufügen einer TAN zu dem Programmierauftrag und Übertragen des Programmierauftrags an das Implantat
- Bei TAN-Listen mit Indices Vergleich des im Implantat gespeicherten, zu dem vom Implantat zuletzt bestimmten Index gehörenden TAN mit der im Programmierauftrag enthaltenen TAN
- Ausführen des Programmierauftrags, falls die TAN identisch sind, oder
- Ignorieren oder Löschen des Programmierauftrags, falls die TAN nicht identisch sind.
- Rückmelden von Erfolg oder Misserfolg der Ausführung des Programmierauftrags.
